# EUROPEAN PATENT APPLICATION

(11) **EP 0 542 249 A2**
(43) Date of publication of application: **19.05.1993**
(21) Application number: 92119346.2
(22) Date of filing: 12.11.1992
(51) Int. Cl.: C12P 19/26

(54) **Method for preparing chitosan**

(30) Priority: 13.11.1991 JP 297435/91
(71) Applicant: SHIN-ETSU CHEMICAL CO., LTD., Chiyoda-ku Tokyo 100 (JP)
(72) Inventor: Takiguchi, Yasuyuki, c/o CORPORATE RESEARCH CENTER, Takatsu-ku, Kawasaki-shi, Kanagawa 213 (JP); Chiba, Tohru, c/o CORPORATE RESEARCH CENTER, Takatsu-ku, Kawasaki-shi, Kanagawa 213 (JP)
(74) Representative: Bühling, Gerhard, Dipl.-Chem.

(57) **Abstract**

A method for preparing chitosan comprises the steps of adding an aqueous organic acid solution having a concentration ranging from 0.5 to 10 % (w/v) to alkali-insoluble substances obtained by treating cultured filamentous fungi with a hot aqueous alkali solution and stirring and mixing the dispersion while heating it at a temperature ranging from 50 to 100 °C to extract chitosan. The method for preparing chitosan makes it possible to provide chitosan in a high productivity. Chitosan is an important biomass and may be used in various fields such as a flocculating agent for polymers, an immobilization support and porous beads.

## Description

The present invention relates to a method for preparing chitosan through cultivation of microorganisms and more particularly to a process for extracting chitosan.

Chitosan has attracted special interest recently as a biomass second to cellulose. It has been anticipated that chitosan can be used in various fields such as flocculating agents for polymers, immobilization supports and porous beads.

Chitosan has industrially been prepared by treating cuticle of Crustacea such as crusts of crabs and lobsters or shrimps with an acid and an alkali to perform the decalcification and deproteinization thereof and then deacetylating the isolated chitin with a hot concentrated alkali solution. Wastes discharged from fish factories have presently been used as the starting material, i.e., cuticle of Crustacea, for the preparation of chitosan, but demand exceeds the amount of the waste and there is not enough of such waste as the starting material in this country. The catches of these starting materials such as crabs are greatly influenced by years and seasons and hence the price thereof is likewise greatly influenced by the catches. Moreover, a large amount of waste water generated during the deacetylation process for chitin leads to a great increase in the biochemical oxygen demand (BOD) of the environment and in turn results in environmental pollution.

The inventors of this invention take note of the fact that a large amount of chitosan is included in the cell wall of filamentous fungi belonging to the family Mucoraceae (see S. Bartnicki-Garcia, S. Ann. Rev. Microbiol., 1968, 22, p. 87-108) and found a method for preparing chitosan through cultivation of filamentous fungi. This invention was already filed as a patent application (Japanese Patent Application Serial No. 3-231893). This method comprises the steps of deproteinizing cell bodies of a filamentous fungus obtained by cultivation with a hot diluted alkali solution to give alkali-insoluble substances; dissolving the alkali-insoluble substances in a diluted acid solution such as acetic acid to thus perform extraction of chitosan; removing insolubles remaining in the diluted acid solution through centrifugation; and alkalizing the resulting supernatant liquid with, for instance, sodium hydroxide to precipitate chitosan.

In the method for preparing chitosan through cultivation of filamentous fungi, however, the amount of chitosan produced per unit volume of the culture medium is small and accordingly the chitosan thus prepared is expensive as compared with those prepared from Crustacea. The inventors of this invention have intensively investigated the conditions for cultivation of the filamentous fungi and succeeded in the improvement of the productivity of chitosan in the foregoing patent application. The inventors of this invention have conducted various studies to further improve the productivity of chitosan.

Accordingly, an object of the present invention is to find out optimum conditions for the cultivation of the filamentous fungi and more specifically to provide a method for preparing chitosan which makes it possible to provide chitosan in a high productivity.

The inventors of this invention have investigated factors which affect the yield of the extract obtained by treating alkali-insoluble substances derived from cultured filamentous fungi with a diluted acid, such as extraction temperature, acid concentrations in extraction solvents used and extraction time, as a result have found that the yield of the extract is substantially improved if an elevated extraction temperature is used and thus have completed the present invention.

According to the present invention, there is provided a method for preparing chitosan which comprises the steps of adding a 0.5 to 10 % (w/v) aqueous solution of an organic acid to an alkali-insoluble substances obtained by treating cultivated filamentous fungi with a hot alkaline aqueous solution; and extracting chitosan while maintaining the mixture at a temperature ranging from 50 to 100 °C and stirring the mixture.

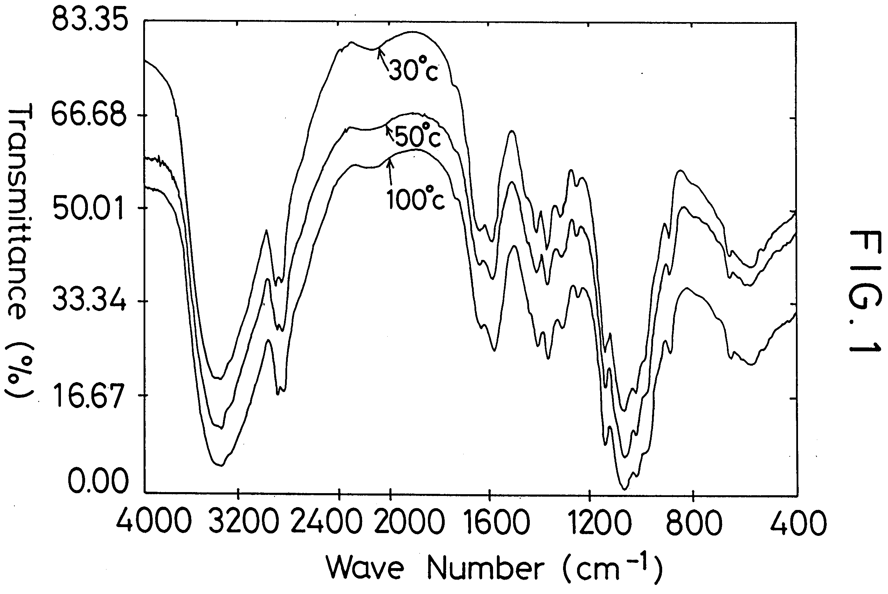

Fig. 1 is a graph showing absorption curves of IR spectroscopic measurement observed at extraction temperatures of 30, 50 and 100 °C , the measurement being performed in Example 2.

The method according to the present invention will hereinafter be explained in more detail.

Among filamentous fungi belonging to the family Mucoraceae, Absidia coerulea is particularly preferred for use in the method of the present invention. This strain is deposited with and managed by Juridical Foundation: Institute of Fermentation (Domicile: 17-85, Juso-honmachi 2-chome, Yodogawa-ku, Osaka-shi, Japan) under the accession number of IFO 5301 and available therefrom.

Cultured filamentous fungi is deproteinized through a heat-treatment in an alkaline aqueous solution to give alkali-insoluble substances. Sodium hydroxide is particularly preferred as an alkali. The cultured filamentous fungi is treated by an aqueous solution of sodium hydroxide while heating the solution at a temperature on the order of 120 °C .

Then an aqueous solution of an organic acid is added to the alkali-insoluble substances to carry out the extraction of chitosan. Examples of organic acids usable in the present invention include acetic acid, lactic acid, butyric acid and oxalic acid with acetic acid being particularly preferred. It is sufficient to adjust the concentration of the aqueous organic acid solution to about 0.5 to 10 % (w/v). At this stage, the extraction temperature is adjusted to a range of from 50 to 100 °C and preferably 80 to 100 °C whereby the amount of chitosan extracted is substantially improved.

When chitosan is extracted by treating the alkali-insoluble substances derived from the cultured filamentous fungi with a diluted acid solution, the extractability of chitosan increases as the temperature of the extraction solution is raised by application of heat. The extraction has been conventionally carried out at a temperature on the order of 30 °C . However, the extraction temperature can be raised up to about 100 °C according to the present invention.

The extractability at a temperature of 30 °C is limited to about 20 to 30 % of the alkali-insoluble substances, but it can be increased up to about 70 % by heating the extraction solution to 100 °C during the extraction.

In addition, the viscosity of the extraction solution decreases as the temperature thereof is increased. This makes the operations for separating and recovering chitosan easy.

The method for preparing chitosan according to the present invention can provide a high extractability of chitosan and this accordingly leads to a high productivity of chitosan. If the extraction of chitosan is carried out at a high temperature, the viscosity of the extraction solution is reduced. As a result, chitosan can easily be recovered and hence can be prepared without any difficulty. Moreover, the amount of residues remaining after the extraction of chitosan is substantially decreased and the productivity of chitosan per unit volume of the culture medium is also improved. This clearly results in the substantial reduction of the production cost of chitosan. The quality of chitosan extracted at a high temperature is not different from and almost identical to that of chitosan extracted at a low temperature.

The present invention will hereinafter be explained in more detail with reference to the following non-limitative working Examples.

### Example 1

To a 3 ℓ small-sized fermentor, there was added 2 ℓ of a culture medium (pH 6.0) which comprised 50 g/ℓ of glucose, 25 g/ℓ of peptone, 15 g/ℓ of yeast extract, 15 g/ℓ of malt extract and 10 g/ℓ of MgSO₄·7H₂O. This culture medium was sterilized by exposing it to high pressure steam of 121 °C for 20 minutes, then precultured myceria (100 mℓ) of Absidia coerulea, strain IFO 5301 were inoculated upon the culture medium and cultured at 27 °C , a rate of aeration of 3 ℓ/min and a number of rotation of 300 rpm for 70 hours. After the cultivation, the resulting myceria were washed with water, suspended in a 2 % (w/v) aqueous solution of sodium hydroxide and heated to 121 °C for 60 minutes in an autoclave. The resulting alkali-insoluble substances were washed with water till the wash liquid became neutral, then lyophilized and stored.

To 100 mℓ of a 2 % (w/v) aqueous solution of acetic acid, there were suspended 0.500 g of the powdery alkali-insoluble substances and the suspension was stirred on a magnetic stirrer at 30 °C for one hour to perform extraction of chitosan. The resulting dispersion was centrifuged (12,000Xg, 10 minutes) to separate it into a supernatant and precipitates. The precipitates were again suspended in 100 mℓ of a 2 % (w/v) aqueous solution of acetic acid to perform extraction in the same manner. The first and second supernatants, i.e., extracts each was subjected to filtration under suction to remove insolubles and the pH thereof was adjusted to not less than 8.5 by the addition of a 40 % (w/v) aqueous solution of sodium hydroxide to thus separate chitosan. The resulting chitosan was collected by centrifugation, washed with water till the wash liquid became neutral and then lyophilized. After the secondary extraction, the resulting extraction residue precipitated out was likewise alkalized, washed with water and then lyophilized. These three samples each was further dried in vacuo and then weighed.

Two extracted and lyophilized samples of chitosan were mixed and pulverized in a mortar and a pestle and then classified by passing through a sieve of 100 mesh size and particles which passed therethrough were used as samples for determining the molecular weight and the degree of deacetylation of the resulting chitosan. The molecular weight thereof was determined by the gel permeation chromatography (GPC) method using polyethylene oxide as a reference material. On the other hand, the degree of deacetylation was determined by the colloid titration method. In this case, the conditions for the extraction of chitosan from the alkali-insoluble substances are a concentration of acetic acid solution of 2 % (w/v), a temperature of 30 °C and an extraction time of one hour while stirring. These conditions are hereinafter referred to as standard extraction conditions and this extraction procedure is referred to as "standard extraction".

Then the extraction of chitosan was repeated under the following extraction conditions. A first experiment was performed at an extraction temperature of 80 °C (other conditions were identical to the standard extraction conditions defined above), a second experiment was carried out using a 10 % (w/v) acetic acid aqueous solution (other conditions were identical to the standard extraction conditions) and a third experiment was carried out under the standard extraction conditions except that the extraction was carried out for 8 hours. After the extraction, each extract was treated in the same manner used above to give samples for the determination of the molecular weight of chitosan and the degree of deacetylation thereof. The results thus obtained are summarized in the following Table 1. The highest extractability of chitosan could be achieved at an extraction temperature of 80 °C . More specifically, the yield of chitosan at that temperature was 0.1791 g (starting from 0.5003 g of the alkali-insoluble substances), while that achieved by the standard extraction was 0.1119 g. The yield is thus 1.6 time greater than that achieved in the standard extraction. The degrees of deacetylation of chitosan were 75.7 % for the standard extraction and 80.1 % for the first experiment, the molecular weights thereof were 390,000 and 352,000 respectively. These results clearly indicate that the quality of the chitosan extracted at a temperature of 80 °C was almost identical to that of the chitosan obtained through the standard extraction.

**Table 1**

| | Standard | 1st Ex. | 2nd Ex. | 3rd Ex. |
|---|---|---|---|---|
| AIM (g) | 0.5006 | 0.5003 | 0.5003 | 0.5001 |
| First Extraction (g) | 0.1058 | 0.1517 | 0.1116 | 0.1133 |
| Second Extraction (g) | 0.0061 | 0.0274 | 0.0118 | 0.0017 |
| Total Extraction (g) | 0.1119 | 0.1791 | 0.1234 | 0.1150 |
| Extraction Residue (g) | 0.3375 | 0.2852 | 0.3368 | 0.3146 |
| a/AIM | 21.1% | 30.3% | 22.3% | 22.7% |
| b/AIM | 1.2% | 5.5% | 2.4% | 0.3% |
| c/AIM | 22.4% | 35.8% | 24.7% | 23.0% |
| d/AIM | 89.8% | 92.8% | 92.0% | 85.9% |
| Degree of Deacetylation | 75.7% | 80.1% | 72.4% | 81.1% |
| Molecular Weight | 390000 | 352000 | 428000 | 472000 |
| Degree of Non-uniformity | 2.73 | 2.90 | 2.92 | 3.01 |
| AIM: alkali-insoluble substances a: First Extraction; b: Second Extraction c: Total Extraction d: Total Extraction + Extraction Residue | | | | |

### Example 2

To 100 mℓ of a 2 % (w/v) aqueous acetic acid solution, there was suspended 0.500 g of alkali-insoluble substances prepared in the same manner used in Example 1, the resulting suspension was extracted in the same manner used in Example 1 at an extraction temperature of 30, 50, 80 or 100 °C to give samples of chitosan. The extractability, molecular weight and degree of deacetylation of each sample were determined in the same manner used in Example 1. In addition, the viscosity of the extract was determined using an E-type viscometer (available from Tokyo Keiki Co., Ltd.). The results thus obtained are listed in the following Table 2.

As seen from the data listed in Table 2, the extractability increases as the extraction temperature is increased. For instance, the extractability observed at an extraction temperature of 100 °C is about 2.4 times greater than that observed at an extraction temperature of 30 °C . The molecular weight of chitosan slightly increases as the extraction temperature increases, but the extraction temperature only slightly affects the degree of deacetylation. Further, the viscosity of the extract is lowered as the temperature increases. For instance, the viscosity thereof observed at 100 °C was reduced to 1/5 time that observed at 30 °C .

The chitosans obtained at extraction temperature of 30, 50 and 100 °C were analyzed by IR spectrometry and the results are shown in Fig. 1. As seen from Fig. 1, absorption spectra of these chitosan are quite similar to each other and thus it can be concluded that these chitosans are approximately identical.

**Table 2**

| | Extraction Temperature | | | |
|---|---|---|---|---|
| | 30 °C | 50 °C | 80 °C | 100°C |
| AIM (g) | 0.501 | 0.501 | 0.502 | 0.501 |
| First Extraction (g) | 0.112 | 0.153 | 0.227 | 0.283 |
| Second Extraction (g) | 0.031 | 0.039 | 0.053 | 0.062 |
| Total Extraction (g) | 0.143 | 0.192 | 0.281 | 0.345 |
| Extraction Residue (g) | 0.265 | 0.221 | 0.137 | 0.081 |
| a/AIM | 22.4% | 30.5% | 45.2% | 56.5% |
| b/AIM | 6.2% | 7.8% | 10.6% | 12.4% |
| c/AIM | 28.5% | 38.3% | 55.8% | 68.9% |
| d/AIM | 81.4% | 82.4% | 83.1% | 85.0% |
| Degree of Deacetylation | 86.1% | 91.7% | 90.2% | 91.0% |
| Molecular Weight | 286000 | 340000 | 355000 | 330000 |
| Degree of Non-uniformity | 2.24 | 2.15 | 2.75 | 3.00 |
| Viscosity of Extract | 10 cP | 6 cP | 4 cP | 2 cP |
| AIM: alkali-insoluble substances a: First Extraction; b: Second Extraction c: Total Extraction d: Total Extraction + Extraction Residue | | | | |

### Example 3

Samples of chitosan were prepared by repeating the procedures used in Example 1 except that, among the standard extraction conditions, various kinds of aqueous solutions detailed below were used for the extraction of chitosan and that the extraction temperature was set at 80 °C , using 0.500 g of alkali-insoluble substances obtained in the same manner used in Example 1. The aqueous solutions used were a 2 % (w/v) acetic acid aqueous solution (Soln. 1), a 2 % (w/v) lactic acid aqueous solution (Soln. 2), a 0.01 N hydrochloric acid aqueous solution (Soln. 3), a 0.01 N sulfuric acid aqueous solution (Soln. 4) and a 0.1 N sulfuric acid aqueous solution (Soln. 5). The extractability, molecular weight and degree of deacetylation of each sample were determined in the same manner used in Example 1. The results thus obtained are summarized in the following Table 3.

**Table 3**

| | Solution Used for Extraction | | | | |
|---|---|---|---|---|---|
| | Soln.1 | Soln.2 | Soln.3 | Soln.4 | Soln.5 |
| AIM (g) | 0.502 | 0.501 | 0.504 | 0.501 | 0.500 |
| First Extraction (g) | 0.227 | 0.192 | 0.044 | 0.025 | 0.006 |
| Second Extraction (g) | 0.053 | 0.062 | 0.008 | 0.005 | 0.001 |
| Total Extraction (g) | 0.281 | 0.254 | 0.052 | 0.029 | 0.007 |
| Extraction Residue (g) | 0.137 | 0.104 | 0.260 | 0.407 | 0.418 |
| a/AIM | 45.2% | 38.2% | 8.7% | 4.9% | 1.2% |
| b/AIM | 6.7% | 12.4% | 1.6% | 1.0% | 0.2% |
| c/AIM | 55.8% | 50.7% | 10.3% | 5.9% | 1.5% |
| d/AIM | 83.1% | 71.4% | 61.8% | 87.3% | 85.0% |
| Degree of Deacetylation | 86.1% | 95.6% | | | |
| Molecular Weight | 355000 | 412000 | 185000 | 131000 | |
| Degree of Non-uniformity | 2.75 | 3.18 | 2.39 | 2.04 | |
| AIM: alkali-insoluble substances a: First Extraction; b: Second Extraction c: Total Extraction d: Total Extraction + Extraction Residue | | | | | |

The extractabilities achieved by inorganic acids solutions, i.e., hydrochloric acid and sulfuric acid solutions (Solns. 3 to 5) are on the order of 10 to 20 % of that achieved by organic acid solutions, i.e., acetic acid and lactic acid solutions (Solns. 1 and 2). In addition, when the sulfuric acid concentration was increased from 0.01 N to 0.1 N, the extractability of chitosan was reduced. In case of extraction with inorganic acid solutions, the amount of chitosan extracted was very small and, therefore, the degree of deacetylation thereof could not be determined. The molecular weights of chitosans recovered through the extraction with inorganic acids are in the order of 1/2 to 1/3 time those of the chitosans recovered through the extraction with the organic acid solutions. It would be assumed that the chitosans are hydrolyzed since they are heated in the presence of strong acids.

A method for preparing chitosan comprises the steps of adding an aqueous organic acid solution having a concentration ranging from 0.5 to 10 % (w/v) to alkali-insoluble substances obtained by treating cultured filamentous fungi with a hot aqueous alkali solution and stirring and mixing the dispersion while heating it at a temperature ranging from 50 to 100 °C to extract chitosan. The method for preparing chitosan makes it possible to provide chitosan in a high productivity. Chitosan is an important biomass and may be used in various fields such as a flocculating agent for polymers, an immobilization support and porous beads.

## Claims

1. A method for preparing chitosan comprising the steps of adding an aqueous organic acid solution having a concentration ranging from 0.5 to 10 % (w/v) to alkali-insoluble substances obtained by treating cultured filamentous fungi with a hot aqueous alkali solution and stirring and mixing the dispersion while heating it at a temperature ranging from 50 to 100 °C to extract chitosan.

2. The method for preparing chitosan according to claim 1 wherein the filamentous fungus is a filamentous fungus belonging to the family Mucoraceae.

3. The method for preparing chitosan according to claim 1 wherein the hot alkali solution is an aqueous sodium hydroxide solution.

4. The method for preparing chitosan according to claim 1 wherein the aqueous organic acid solution is a member selected from the group consisting of an acetic acid aqueous solution, a lactic acid aqueous solution, a butyric acid aqueous solution and an oxalic acid aqueous solution.
